# EUROPEAN PATENT APPLICATION

(11) **EP 4 082 532 A1**
(43) Date of publication of application: **02.11.2022**
(21) Application number: 20904461.9
(22) Date of filing: 22.12.2020
(51) Int. Cl.: A61K 9/20, A61K 31/4985, A61K 31/7034, A61K 31/155, A61P 3/10

(54) **COMPLEX FORMULATION COMPRISING SITAGLIPTIN AND DAPAGLIFLOZIN, AND PREPARATION METHOD THEREFOR**

(30) Priority: 24.12.2019 KR 20190174277; 30.03.2020 KR 20200038569
(71) Applicant: Hanmi Pharm. Co., Ltd., Hwaseong-si, Gyeonggi-do 18536 (KR)
(72) Inventor: CHANG, Seung Hun, Anyang-si, Gyeonggi-do 14045 (KR); LEE, Kwang Young, Yongin-si, Gyeonggi-do 16830 (KR); KIM, Jae Ho, Gwacheon-si, Gyeonggi-do 13835 (KR); CHO, Jung Hyun, Hwaseong-si, Gyeonggi-do 18477 (KR); KIM, Yong Il, Gwacheon-si, Gyeonggi-do 13835 (KR)
(74) Representative: Brevalex
(86) International application number: PCT/KR2020/018871
(87) International publication number: WO 2021/133023

(57) **Abstract**

Provided are a composite formulation including sitagliptin and dapagliflozin and a method of preparing the same.

## Description

### TECHNICAL FIELD

The present application relates to a composite formulation including sitagliptin and dapagliflozin and a preparation method therefor, and more particularly, to a composite formulation which has excellent stability, productivity, dissolution rate, and compounding compatibility and can be formed to be smaller, and a preparation method therefor.

### BACKGROUND ART

In general, type 2 diabetes patients are accompanied by being overweight, abdominal obesity, and high blood pressure, and thus diabetes is known as a disease that causes secondary chronic diseases or metabolic syndromes, such as hypertension, hyperlipidemia, myocardial infection, and stroke. According to medical guidelines of the Korean Diabetes Association, combined drug therapy is actively recommended to enhance improvement of symptoms. In particular, the combined use of DPP-4 inhibitor drugs and SGLT-2 inhibitor drugs has recently been proven by academia to have excellent efficacy in the treatment of diabetes, and even three-drug treatment with metformin is also under research.

Sitagliptin (Product name: JANUVIA tablet) is a dipeptidyl peptidase-4 (DPP-4) inhibitor drug and its compound name is (R)-3-amino-1-(3-(trifluoromethyl)-5,6-dihydro-[1,2,4]triazolo[4,3-a]pyrazin-7(8H)-yl)-4-(2,4,5-trifluorophenyl)butan-1-one. Sitagliptin regulates blood sugar by inhibiting the breakdown of gastrointestinal hormones called incretins to enable the incretins, which regulate insulin and glucagon, to function well in the body. It is known that when sitagliptin is orally administered to a patient with type 2 diabetes, HbA1c levels are significantly reduced, and fasting blood sugar and postprandial blood sugar secretion are reduced.

Dapagliflozin (Product name: FORXIGA tablet) is a sodium-glucose linked transporter 2 (SGLT-2) inhibitor drug, and its compound name is (2S,3R,4R,5S,6R)-2-[4-chloro-3-(4-ethoxybenzyl)phenyl]-6-(hydroxymethyl)tetrahydro-2H-pyran-3,4,5-triol. Dapagliflozin selectively inhibits SGLT2 in the kidneys and increases the excretion of glucose in the urine, thereby improving insulin sensitivity and delaying the onset of diabetic complications, and thus normalizing plasma glucose levels. Dapagliflozin is currently sold on the market by the original developer AstraZeneca AB, in the form of tablets (FORXIGA tablet) including dapagliflozin propylene glycol hydrate as an active ingredient.

Sitagliptin and dapagliflozin have the main effect of blood sugar reduction without a risk of low blood sugar. In addition, sitagliptin has the effects of protecting pancreatic beta cells and increasing GLP-1, and dapagliflozin has weight loss and blood pressure reduction effects, and has also been introduced with clinical results wherein a combination of two active ingredients has a synergistic effect. In addition, in the case of diabetic patients, as diabetes progresses, it becomes difficult to control blood sugar, resulting in complications. Specifically, elderly diabetic patients are more likely to suffer from high blood pressure, obesity, and hyperlipidemia. Due to these characteristics of diabetic patients, medication compliance is a very crucial factor, a reduction in medication compliance not only lowers a patient's quality of life, but also reduces a patient's treatment rate, increasing personal medical expenses and worsening insurance finances. Therefore, it is necessary to develop a composite formulation including sitagliptin and dapagliflozin.

However, the development of a composite formulation has not yet been attempted due to numerous accumulated problems, such as physical properties of active pharmaceutical ingredients (API), tablet size, and the like. For sitagliptin as an active ingredient, there are problems that the active ingredient is contained in a large amount per tablet and punch sticking is liable to occur during a production process because the active ingredient has viscosity. In addition, dapagliflozin as an active ingredient has poor productivity because it has a large volume even in a small amount due to low density, and has a problem of difficulty ensuring formulation uniformity because layer separation from other active ingredients and excipients, and agglomeration due to the aggregating characteristics of the active ingredients, are more likely to occur.

Furthermore, in consideration of the total mass of each of the representative tablets including sitagliptin and dapagliflozin, respectively, as active ingredients (a total amount of JANUVIA tablet 100 mg, including 100 mg of sitagliptin, is about 416.1 mg and a total amount of FORXIGA tablet 10 mg, including 10 mg of dapagliflozin, is about 260 mg), a composite formulation including the active ingredients may have a problem of reduction in medication compliance due to an increase in the total amount including the active ingredients and excipients and a consequential increase in tablet size. In other cases, when the total amount of the composite formulation and the tablet size are excessively reduced, due to an increased ratio of the active ingredients to excipient, it may be difficult to overcome the physical properties of the active ingredients during product production. In addition, in the composite formulation development field, it is one of the crucial and difficult issues to screen excipients to satisfy compounding compatibility of both of the different active ingredients included in the composite formulation. Furthermore, the time (Tmax) required for the drug to reach a maximum concentration in plasma is about 1 hour for both sitagliptin and dapagliflozin (1-4 hr for sitagliptin and 1-2 hr for dapagliflozin), and there is a need to develop a composite formulation including the two ingredients and at the same time having an excellent dissolution rate.

### [Prior art document]

KR 10-2016-0111237

### DESCRIPTION OF EMBODIMENTS

### TECHNICAL PROBLEM

According to an aspect of the present disclosure, provided are sitagliptin or a pharmaceutically acceptable salt thereof, or a hydrate thereof, and dapagliflozin or a pharmaceutically acceptable salt thereof, or a hydrate thereof which have excellent stability, productivity, dissolution properties, and compounding compatibility, while at the same time enabling a reduction in dosage size and consequently increasing medication compliance.

According to another aspect, provided is a method of preparing the composite formulation.

Other objects and advantages of the present application will become more apparent from the following detailed description in conjunction with the appended claims. Contents not described in this specification can be sufficiently recognized and inferred by a person skilled in the art within the technical field of the present application or a similar technical field, and thus description thereof is omitted.

### SOLUTION TO PROBLEM

According to an aspect, provided is a composite formulation including:
sitagliptin or a pharmaceutically acceptable salt thereof, or a hydrate thereof, and
dapagliflozin or a pharmaceutically acceptable salt thereof, or a hydrate thereof,
wherein the composite formulation includes, as a lubricant, sodium stearyl fumarate, magnesium stearate, or a combination thereof.

According to another aspect, provided is a method of preparing the composite formulation according to the one aspect, the method including:
preparing a mixture portion including sitagliptin or a pharmaceutically acceptable salt thereof, or a hydrate thereof, and dapagliflozin or a pharmaceutically acceptable salt thereof, or a hydrate thereof, an excipient, and a lubricant;
granulating the mixture portion; and
further adding a lubricant to the granulated material and mixing together.

According to still another aspect, provided is a method of preparing the composite formulation according to the one aspect, the method including:
preparing a first mixture portion including sitagliptin or a pharmaceutically acceptable salt thereof, or a hydrate thereof, an excipient, and a lubricant;
preparing a second mixture portion including dapagliflozin or a pharmaceutically acceptable salt thereof, or a hydrate thereof, an excipient, and a lubricant;
separately granulating the first mixture portion and/or the second mixture portion; and
mixing obtained granulated material with a non-granulated mixture portion and an additional lubricant.

### ADVANTAGEOUS EFFECTS OF DISCLOSURE

The composite formulation including sitagliptin and dapagliflozin, according to an aspect, may have excellent stability, productivity, dissolution properties, and compounding compatibility, and at the same time, and enables to reduce the size of the dosage form, thus increasing medication compliance. The preparation method according to an aspect may improve pharmaceutical properties of the composite formulation such as flowability and tableting properties, thereby enhancing manufacturing productivity.

### BRIEF DESCRIPTION OF DRAWINGS

FIG. 1 shows photographs taken of compacted flakes and tablets prepared from a composite formulation of sitagliptin and dapagliflozin with or without magnesium stearate.
FIG. 2 shows images of a tablet prepared by mixing and tableting sitagliptin and lactose, and a tablet prepared by mixing and tableting sitagliptin and an excipient other than lactose.
FIG. 3 shows images showing the appearances of the tablets of Example 5 and Comparative Example 5 after tableting, prepared with a different amount of sodium stearyl fumarate (PRUV^{®}), and the results of measuring the time required for discharging granules during tableting of each tablet.
FIG. 4 is a graph showing results of a sitagliptin dissolution test of Examples 5-8 and Comparative Examples 6 and 8.
FIG. 5 is a graph showing results of a dapagliflozin dissolution test of Examples 5-8 and Comparative Examples 6 and 8.
FIG. 6 is a process chart illustrating a process of preparing composite tablets of Examples 9-12, in which the numbers in FIG. 6 indicate the numbers of the ingredients shown in Table 18 (Examples 9 and 10) and Table 19 (Examples 11 and 12).
FIG. 7 is a graph showing results of measuring particle size distribution (mesh profile) using granules prepared in Examples 11 and 12.
FIG. 8 shows photographs taken of (a) JANUVIA 100 mg (100 mg of sitagliptin), (b) FORXIGA 10 mg (12.3 mg of dapagliflozin propanediol hydrate), and (c) a composite tablet prepared in Example 13.

### MODE OF DISCLOSURE

Hereinafter, the present invention will be described in more detail.

All technical terms used herein have the same meaning as commonly understood by one of ordinary skill in the art, unless defined otherwise. In addition, although preferred methods or samples are described herein, similar or equivalent ones also fall within the scope of the present specification. In addition, the numerical values described herein are considered to include the meaning of "about" even if not specified. The contents of all publications incorporated herein by reference are hereby incorporated by reference in their entirety. As used herein, the term "about" means that the referenced value may vary to some extent. For example, the value may vary by 10%, 5%, 2%, or 1%. For example, "about 5" is meant to include any value between 4.5 and 5.5, between 4.75 and 5.25, or between 4.9 and 5.1, or between 4.95 and 5.05. As used herein, the terms "has", "may have", "comprises", or "may include" indicate the presence of a corresponding feature (e.g., a numerical value or a component such as an ingredient), and does not exclude the presence of additional features.

An aspect provides a composite formulation including: sitagliptin or a pharmaceutically acceptable salt thereof, or a hydrate thereof; and dapagliflozin or a pharmaceutically acceptable salt thereof, or a hydrate thereof,
wherein the composite formulation includes, as a lubricant, sodium stearyl fumarate, magnesium stearate, or a combination thereof.

In one embodiment, the composite formulation may include sodium stearyl fumarate as a lubricant.

The active ingredient sitagliptin or dapagliflozin may include all of the crystalline forms, hydrates, co-crystals, solvates, salts, diastereomers, or enantiomers thereof.

The pharmaceutically acceptable salt thereof refers to any pharmaceutically acceptable salt that may be commonly used in the art.

In one embodiment, the sitagliptin or a pharmaceutically acceptable salt thereof, or a hydrate thereof, may be sitagliptin phosphate hydrate.

In one embodiment, the dapagliflozin or a pharmaceutically acceptable salt thereof, or a hydrate thereof may be a pharmaceutically acceptable co-crystal of dapagliflozin. In one embodiment, the dapagliflozin or a pharmaceutically acceptable salt thereof may be dapagliflozin L-proline or dapagliflozin propandiol hydrate. In one embodiment, the dapagliflozin or a pharmaceutically acceptable salt thereof may be dapagliflozin L-proline.

In one embodiment, the sitagliptin or a pharmaceutically acceptable salt thereof, or a hydrate thereof may be included in an amount of 10-40% by weight, for example, 25-35% by weight of the total weight of the composite formulation.

In one embodiment, the dapagliflozin or a pharmaceutically acceptable salt thereof, or a hydrate thereof may be included in an amount of 2-10% by weight, for example, 2-6% by weight of the total weight of the composite formulation.

In one embodiment, as the lubricant, sodium stearyl fumarate may be included in an amount of 3-8% by weight based on the total weight of the composite formulation.

Magnesium stearate, which is most commonly used as a lubricant, increases productivity and tableting properties of the composite formulation of the present application, but has been found to be very disadvantageous because it increases the related compounds of the active ingredients over time (Test Examples 1 and 3). In contrast, sodium stearyl fumarate may increase productivity and tableting properties, and may also form a composite formulation stable enough to satisfy the standard levels of related compounds (Test Example 3). The sodium stearyl fumarate may be included in an amount of 3-8% by weight based on the total weight of the composite formulation. When the amount of sodium stearyl fumarate is less than 3% by weight, there is a concern that sufficient stability and productivity may not be ensured. When the amount of sodium stearyl fumarate exceeds 8% by weight, there is a concern about a reduction in dissolution rate of the active ingredients (Test examples 4, 5, and 6).

The composite formulation may include one or more excipients selected from a diluent, a disintegrant, a binder, a lubricant, and a release controlling agent.

The diluent may be selected from the group consisting of, for example, D-mannitol, pregelatinized starch, low-substituted hydroxypropylcellulose (L-HPC), microcrystalline cellulose (MCC), sucrose, sorbitol, xylitol, glucose, and any mixtures thereof, but is not limited thereto.

In one embodiment, the diluent may be selected from the group consisting of D-mannitol, pregelatinized starch, low-substituted hydroxypropyl cellulose (L-HPC), microcrystalline cellulose, and any combination thereof.

The disintegrant may be selected from the group consisting of, for example, crospovidone, sodium cross-linked carboxymethylcellulose (cross-linked CMC Na, C.CMC Na, or croscarmelose sodium), corn starch, calcium carboxymethylcellulose, sodium starch glycolate, low-substituted hydroxypropyl cellulose (L-HPC), and any mixtures thereof, but is not limited thereto. In one embodiment, the disintegrant may be sodium cross-linked carboxymethylcellulose.

The binder may be selected from the group consisting of, for example, sodium carboxymethylcellulose, ethylcellulose, hydroxyethylcellulose, hydroxypropylcellulose, hydroxypropylmethylcellulose, methylcellulose, gelatin, povidone, and any mixtures thereof, but is not limited thereto. In one embodiment, the binder may be hydroxypropylcellulose.

The release control agent may be selected from the group consisting of, for example, hydroxypropylmethylcellulose, hydroxypropyl cellulose, carboxyvinyl polymer, polyvinyl alcohol, xanthan gum, guar gum, carboxymethyl cellulose and its derivatives, methylcellulose and its derivatives, povidone-polyvinylacetate copolymer, and any mixtures thereof, but is not limited thereto. In one embodiment, the release control agent may be hydroxypropyl cellulose.

In one embodiment, the combination formulation may include an excipient selected from among microcrystalline cellulose (MCC), mannitol, pregelatinized starch, low-substituted hydroxypropyl cellulose (L-HPC), crospovidone, cross-linked carboxymethyl cellulose sodium (cross-linked CMC Na), and any mixtures thereof.

The composite formulation may be in the form of a tablet, a capsule, or a granule. In one embodiment, the composite formulation may be a mixed tablet or a double-layer tablet.

In one embodiment, the composite formulation may be in the form of a tablet including compacted granules including the active ingredients and excipients. For the tablet including the compacted granules, it was confirmed that the flowability of the mixture supplied into a cavity during tableting was excellent, and thus productivity was excellent and content uniformity was excellent (Test Example 7), compared to directly compressed tablets.

The composite formulation may include an additional pharmaceutically acceptable excipient. The additional pharmaceutically acceptable excipient may be an ingredient selected from the group consisting of antioxidants, sweeteners, preservatives, coating agents, viscosity control agents, and any combinations thereof.

The composite formulation may be in a form in which the width and length are each about 5-15 mm. The composite formulation may have a thickness of about 3-8 mm. In one embodiment, the composite formulation may be in a form in which the width and length are each about 5-15 mm and the thickness is about 3-8 mm. In one embodiment, the composite formulation may be in a form in which the width is about 10-15 mm, the length is about 5-10 mm, and the thickness is about 3-8 mm. The width of the composite formulation may be, for example, about 10, 11, 12, 13, 14, or 15 mm. The length of the composite formulation may be, for example, about 5, 6, 7, 8, 8, or 10 mm. The thickness of the composite formulation may be, for example, about 3, 4, 5, 6, 7, or 8 mm.

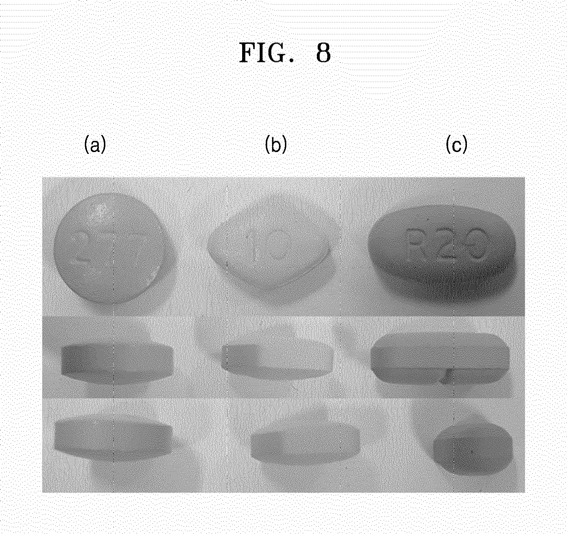

The composite formulation may be a rectangular oval tablet. The composite formulation may be easier to swallow than when taking two tablets respectively containing two active ingredients. In general, when a tablet passes through the narrow area of the throat of the human body after the administration of the drug, the tablet passes through the throat while maintaining the smallest cross-sectional area. At this time, to maintain the small cross-sectional area of the tablet, throat swallowing takes place while maintaining the two smallest variables of the width, length, and thickness of the tablet. For example, FIG. 8 shows images of (a) JANUVIA tablets (100 mg of sitagliptin), (b) FORXIGA tablets (12.3 mg of dapagliflozin propanediol hydrate), and (c) the tablets prepared in Example 13, showing the appearance of each tablet expected to be aligned upon swallowing. In the composite formulation including the two active ingredients of sitagliptin and dapagliflozin, according to one embodiment, the size and weight of the drug and the cross-sectional area upon swallowing may be reduced, and the convenience of medication for patients who feel uncomfortable when swallowing may be increased, compared to a case of simultaneously taking individual tablets containing each active ingredient as a single ingredient.

The composite formulation may further include one or more antidiabetic agents. For example, the composite formulation may further include metformin or a pharmaceutically acceptable salt thereof. In one embodiment, the composite formulation may be a three-drug composite preparation including sitagliptin, dapagliflozin, and metformin.

Another aspect provides a method of preparing the composite formulation.

In one embodiment, the preparation method may include:
preparing a mixture portion including sitagliptin or a pharmaceutically acceptable salt thereof, or a hydrate thereof, and dapagliflozin or a pharmaceutically acceptable salt thereof, or a hydrate thereof, an excipient, and a lubricant;
granulating the mixture portion; and
further adding a lubricant to the granulated material and mixing together.

In another embodiment, the preparation method may include:
preparing a first mixture portion including sitagliptin or a pharmaceutically acceptable salt thereof, or a hydrate thereof, an excipient, and a lubricant;
preparing a second mixture portion including dapagliflozin or a pharmaceutically acceptable salt thereof, or a hydrate thereof, an excipient, and a lubricant;
separately granulating the first mixture portion and/or the second mixture portion; and
mixing obtained granulated material with a non-granulated mixture portion and an additional lubricant.

In one embodiment, the granulation may be performed by a dry granulation method. The dry granulation method may include forming compacted flakes by using a roller compactor.

In one embodiment, the preparation method may further include tableting a mixture mixed with an additional lubricant.

One or more embodiments of the present disclosure will now be described in detail with reference to the following examples. However, these examples are only for illustrative purposes and are not intended to limit the scope of the one or more embodiments of the present disclosure.

### Test methods

In the following test examples, related compound analysis, dissolution analysis, and content analysis conditions were carried out in the following manners.

### 1. Related compound analysis conditions [HPLC analysis]

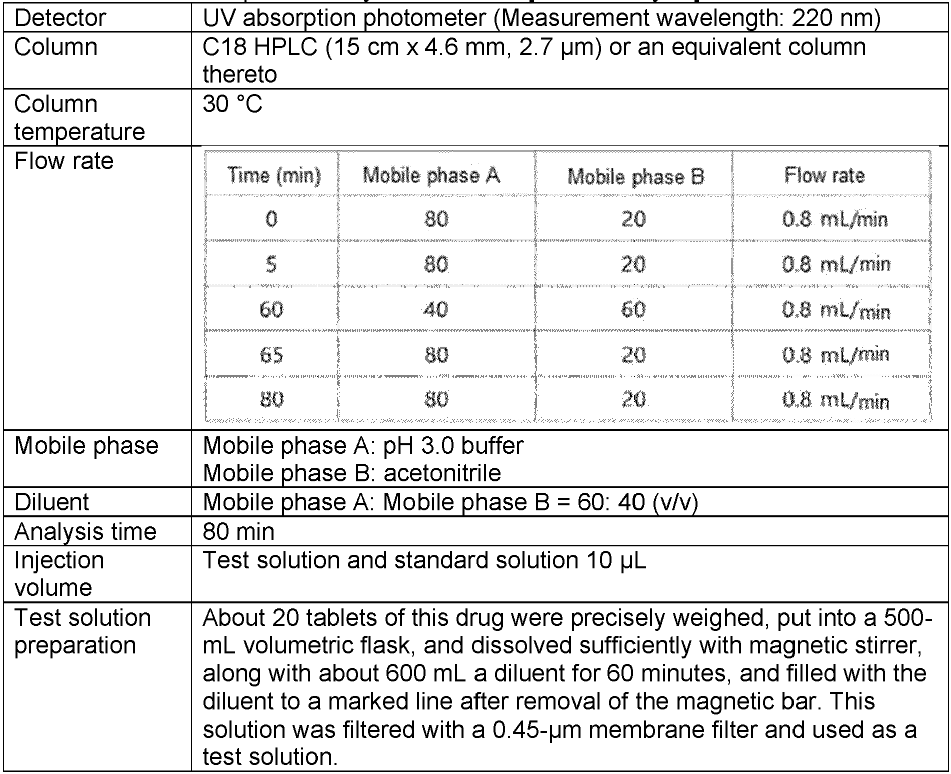

### 2. Dissolution analysis conditions [HPLC analysis]

| Detector | UV absorption photometer (measurement analysis: 205 nm) |
|---|---|
| Column | C18 HPLC (15 cm × 4.6 mm, 5 µm) or an equivalent column thereto |
| Column temperature | 25 °C |
| Flow rate | 1.0 mL/min |
| Mobile phase | pH 7.2 buffer: acetonitrile = 3:2 (v/v) |
| Diluent | Same as mobile phase |
| Analysis time | 30 min |
| Injection volume | Test solution and standard solution 20 µL |
| Test solution preparation | 10 mL of dissolution solution was taken at a sampling time for analysis after dissolution starts and filtered with a 0.45-µm membrane filter to use the filtrate as a test solution. |
| Dissolution apparatus | Dissolution test method 2 (Paddle method) of the general test methods in the Korean Pharmacopoeia |
| Dissolution test solution | 900 mL of pH 1.2 solution (Solution 1 of the dissolution test in the Korean Pharmacopoeia) |
| Dissolution apparatus temperature | 37 °C ± 0.5 °C |
| Dissolution apparatus rotation number | 75 ± 2 rpm |

### 3. Amount analysis conditions [HPLC analysis]

| Detector | UV absorption photometer (Measurement wavelength: 205 nm) |
|---|---|
| Column | C18 HPLC (15 cm × 4.6 mm, 5 µm) or an equivalent column thereto |
| Column temperature | 25 °C |
| Flow rate | 1.0 mL/min |
| Mobile phase | pH 7.2 buffer: acetonitrile = 3:2 (v/v) |
| Diluent | Same as mobile phase |
| Analysis time | 30 min |
| Injection volume | Test solution and standard solution 20 µL |
| Test solution preparation | 5T tablets were put into a 500-mL volumetric flask, filled with about 300 mL of a diluent, and subjected to ultrasonication for extraction for about 1 hour. This volumetric flask was cooled down at room temperature and filled with the diluent solution to a marked line. 4 ml of the solution in the volumetric flask was put into a 50-mL |
| | volumetric flask and then filled with the diluent to a marked line. This solution was filtered with a 0.45-µm membrane filter to use the filtrate as a test solution. |

### Test example 1: Related compound test with lubricants

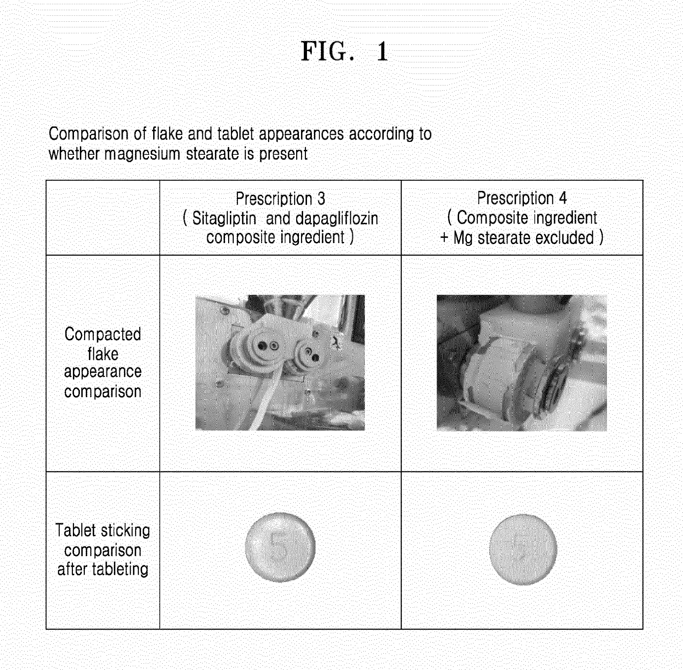

The stability of the composite formulation when commonly used magnesium stearate was used as a lubricant was tested. With prescriptions according to Table 1, tablets were prepared according to the following [Sample preparation method]. Then, related compounds were measured for each sample (Tables 1 to 3), the appearances of pressed flakes and tablets according to the presence or absence of magnesium stearate were compared (FIG. 1). FIG. 1 illustrates images of pressed flakes and tablets according to the presence or absence of magnesium stearate.

### [Sample preparation method]

(1) Weighing: Each ingredient was weighed to be an amount of 1000T.
(2) Sieving: All excipients, except for a lubricant to be added in final mixing, were sieved through a 30-mesh sieve.
(3) Mixing: Powder having passed through the sieve was mixed with a Bin mixer at 17 rpm for 30 min.
(4) Compacting: Pressed flakes were formed using a roller compactor at a roll rpm of 3.0 and a screw rpm of 35.0 at a hydraulic pressure of 2.5 Mpa.
(5) Sizing: The flakes prepared in step (4) were sized with a 20-mesh using Oscillator.
(6) Final mixing: The resulting product prepared in step (5) and the remaining final mixing lubricant were added and mixed with a bin mixer at 17 rpm for 5 min.
(7) Tableting: Tableting was performed with a tablet hardness of 10-12 kp using an AutoTab-200TR (Ichihachi Seiki Co., Ltd, Japan) with a circular punch having a diameter of 8.0 mm.

**[Table 1]**

| | | | | | |
|---|---|---|---|---|---|
| Process | Excipient | Prescription 1 (Single ingredient of sitagliptin) | Prescription 2 (Single ingredient of dapagliflozin) | Prescription 3 (Composite ingredient of sitagliptin and dapagliflozin) | Prescription 4 (Composite ingredient + Mg stearate excluded) |
| Weighing ↓ Sieving and mixing ↓ Compacting ↓ Sizing | Sitagliptin phosphate hydrate | 128.5 | - | 128.5 | |
| | Dapagliflozin L-proline | - | 15.6 | 15.6 | |
| | Microcrystalline cellulose (MCC) | 153.2 | 266.1 | 137.6 | 145.6 |
| | D-mannitol | 102.0 | | | |
| | Croscarmelose sodium | 8.3 | | | |
| | Magnesium stearate | 4.0 | | | 0.0 |
| Final mixing | Magnesium stearate | 4.0 | | | 0.0 |
| Total (mg) | | 400.0 | | | 400.0 |

**[Table 2] Related compound results under accelerated conditions**

| Results of total related compound of sitagliptin (%) [Total related compound standard level: 0.2% or less] | | | | |
|---|---|---|---|---|
| | Prescription 1 (Single ingredient of sitagliptin) | Prescription 2 (Single ingredient of dapagliflozin) | Prescription 3 (Composite ingredient of sitagliptin and dapagliflozin) | Prescription 4 (Composite ingredient + Mg stearate excluded) |
| Initial | 0.01 | - | 0.01 | 0.01 |
| Acceleratio n 1M | 0.03 | - | 0.09 | 0.04 |
| Acceleratio n 3M | 0.08 | - | **0.25** | 0.09 |

**[Table 3] Related compound results under accelerated conditions**

| Results of total related compound of dapagliflozin (%) [Total related compound standard level: 2.0% or less] | | | | |
|---|---|---|---|---|
| | Prescription 1 (Single ingredient of sitagliptin) | Prescription 2 (Single ingredient of dapagliflozin) | Prescription 3 (Composite ingredient of sitagliptin and dapagliflozin) | Prescription 4 (Composite ingredient + Mg stearate excluded) |
| Initial | | 0.03 | 0.05 | 0.04 |
| Acceleration 1M | | 0.20 | 0.78 | 0.22 |
| Acceleration 3M | | 0.58 | 2.21 | 0.63 |

According to the above experimental results, when magnesium stearate was used as a lubricant in the mixed granules including sitagliptin and dapagliflozin together, related compounds were greatly increased under accelerated storage conditions. In addition, when magnesium stearate was excluded, there was a decrease in productivity, due to the issue of adhering to the punch and production equipment during the granulation process and product tableting. Therefore, it was confirmed that, though a lubricant is necessary for productivity and tableting properties, magnesium stearate lowers the stability of the active ingredients and is not appropriate.

### Test example 2: Related compound test with excipients

### Preparation example 1

To evaluate stability when the two active ingredients of sitagliptin and dapagliflozin coexist, tablets including 128.5 mg of sitagliptin phosphate hydrate (100 mg of sitagliptin) and 15.6 mg of dapagliflozin L-proline (10 mg of dapagliflozin) were prepared through compacting. Using a AutoTab-200TR (Ichihachi Seiki Co., Ltd, Japan) tableting apparatus, flakes were formed, and the amounts of related compounds generated under accelerated conditions (40 °C/75 % RH) for 1 month/2 months were measured to confirm stability.

### Preparation example 2

To find out the compounding compatibility of the active ingredient sitagliptin with excipients, sitagliptin was mixed with different types of excipients. The active ingredient sitagliptin and each excipient were sieved through a 20-mesh sieve, and then mixed with a Tubular mixer for 30 minutes. Then, the other active ingredient dapagliflozin was added thereto, mixed, and compacted to form tablets. Using a AutoTab-200TR (Ichihachi Seiki Co., Ltd, Japan) tableting apparatus, flakes were formed, and the amounts of related compounds generated under accelerated conditions (40 °C/75% RH) for 1 month/2 months were measured to confirm stability.

### Preparation example 3

To find out the compound compatibility of the active ingredient dapagliflozin with excipients, dapagliflozin was mixed with each different type of excipient. The active ingredient dapagliflozin and each excipient were sieved through a 20-mesh sieve, and then mixed with a Tubular mixer for 30 minutes. Then, the other active ingredient sitagliptin was added thereto, mixed, and compacted to form tablets. Using a AutoTab-200TR (Ichihachi Seiki Co., Ltd, Japan) tableting apparatus, flakes were formed, and the amounts of related compounds generated under accelerated conditions (40 °C/75 % RH) for 1 month/2 months were measured to confirm stability.

### Standard level for total related compound content

According to the permitted related compound standards in Korea, currently applied standard levels for total related compound content are 0.2% or less of total related compounds for sitagliptin, and less than 2.0% of total related compounds for dapagliflozin.

**[Table 4] Total related compound content of sitagliptin (%)**

| | | | | | | | | | | |
|---|---|---|---|---|---|---|---|---|---|---|
| Ingredient | API+API | Compounding compatibility of sitagliptin API + excipient (mg) | | | | | | | | |
| Sitagliptin phosphate hydrate | 128.5 mg | 128.5 mg (100 mg of sitagliptin) | | | | | | | | |
| Dapagliflozin L-proline | 15.6 mg | 15.6 mg (10 mg of dapagliflozin) | | | | | | | | |
| | - | 150 mg | 150 mg | 150 mg | 150 mg | 150 mg | 150 mg | 150 mg | 50 mg | 50 mg |
| Excipient | Dapagliflozin | MCC | DCP anhydride | lactose hydrate | mannitol | DCP hydrate | pregelatinized starch | L-HPC | crospovidone | C. CMC Na |
| Initial | 0.01 | 0.01 | 0.01 | 0.01 | 0.01 | 0.02 | 0.01 | 0.01 | 0.02 | 0.01 |
| Acceleration 1 M | 0.02 | 0.02 | 0.02 | 0.14 | 0.03 | 0.17 | 0.05 | 0.02 | 0.04 | 0.02 |
| Acceleration 2M | 0.03 | 0.03 | 0.03 | 0.28 | 0.04 | 0.31 | 0.07 | 0.06 | 0.05 | 0.03 |

The results of the related compound test performed using the tablets prepared in Preparation Example 1 and Preparation Example 2 are shown in Table 4. As shown in Table 4, for sitagliptin, when lactose hydrate or dicalcium phosphate (DCP) hydrate was included as an excipient, the total related compound content was greater than 0.2% for each case, failing to satisfy the standard level.

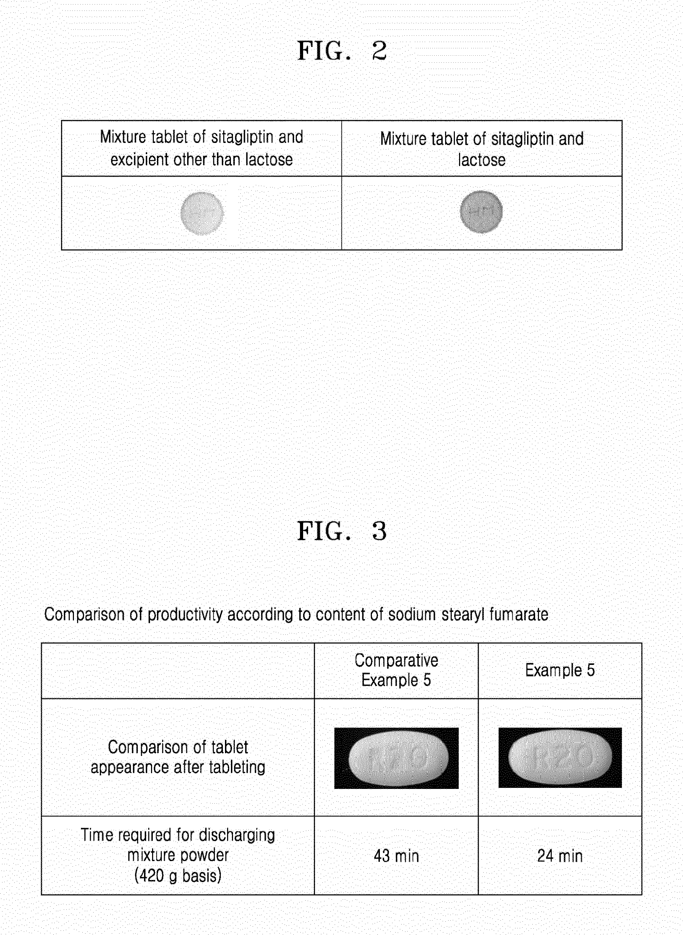

Specifically, when sitagliptin and lactose hydrate were mixed, and the appearance was observed after two months of acceleration, a browning phenomenon occurred due to the Maillard reaction that produces a brown substance at high temperature, confirming that the formulation is not suitable for prescription (FIG. 2). FIG. 2 shows images of a tablet prepared by mixing and tableting sitagliptin and lactose, and a tablet prepared by mixing and tableting sitagliptin and an excipient other than lactose.

**[Table 5] Total related compound content of dapagliflozin (%)**

| | | | | | | | | | | |
|---|---|---|---|---|---|---|---|---|---|---|
| Ingredient | API+API | Compounding compatibility of dapagliflozin API + excipient (mg) | | | | | | | | |
| Dapagliflozin L-proline | 15.6 | 15.6 (10 mg of dapagliflozin) | | | | | | | | |
| Sitagliptin phosphate hydrate | 128.5 mg | 128.5 mg (100 mg of sitagliptin) | | | | | | | | |
| | | 150 mg | 150 mg | 150 mg | 150 mg | 150 mg | 150 mg | 150 mg | 50 mg | 50 mg |
| Excipient | Sitagliptin | MCC | DCP anhydride | lactose hydrate | mannitol | DCP hydrate | pregelatinized starch | L-HPC | crospovidone | C. CMC Na |
| Initial | 0.01 | 0.03 | 0.05 | 0.04 | 0.04 | 0.05 | 0.05 | 0.04 | 0.04 | 0.04 |
| Acceleration 1 M | 0.02 | 0.04 | 1.79 | 0.05 | 0.07 | 0.98 | 0.07 | 0.05 | 0.05 | 0.05 |
| Acceleration 2 M | 0.03 | 0.05 | 3.52 | 0.06 | 0.08 | 1.25 | 0.09 | 0.08 | 0.06 | 0.06 |

The results of the related compound test performed using the tablets prepared in Preparation Example 1 and Preparation Example 3 are shown in Table 5. As shown in Table 5, for dapagliflozin, when dicalcium phosphate (DCP) anhydride was included as an excipient, the total related compound content was greater than 2.0%, failing to satisfy the standard level. In addition, when dicalcium phosphate (DCP) hydrate was included as an excipient, the total related compound content satisfied the standard level, but was higher compared to the other excipients.

### Test example 3: Related compound test with lubricants and excipients

Based on the stability comparison results with respect to each prescription in Test Examples 1 and 2, it was confirmed that it is necessary to choose lubricants or excipients suitable for production and having ensured stability. Accordingly, a stability test was carried out with various types of lubricants and excipients. After completion of the production of tablets, the stability test was carried out under accelerated and stress conditions.

With prescriptions according to Table 6, tablets were prepared according to [Sample preparation method] as follows.

### [Sample preparation method]

(1) Weighing: Each ingredient was weighed to be an amount of 1000T.
(2) Sieving: All excipients and active ingredients, except for a lubricant to be added in final mixing, were sieved through a 30-mesh sieve.
(3) Mixing: Powder having passed through the sieve was mixed with a Bin mixer at 17 rpm for 30 min.
(4) Compacting: Pressed flakes were formed using a roller compactor at a roll rpm of 3.0 and a screw rpm of 35.0 at a hydraulic pressure of 2.5 Mpa.
(5) Sizing: The flakes prepared in step (4) were sized using Oscillator with a 20-mesh.
(6) Final mixing: The resulting product prepared in step (5) and the remaining final mixing lubricant were added and mixed with a Bin mixer at 17 rpm for 5 min.
(7) Tableting: Tableting was performed with a tablet hardness of 12-14kp using an AutoTab-200TR (Ichihachi Seiki Co., Ltd, Japan) with a rectangular punch having a width of 12.8 mm and a length of 7.0 mm.

**[Table 6] Prescription with different excipients/lubricants [units: mg]**

| Process | No . | Excipient | Exampl e 1 | Exampl e 2 | Exampl e 3 | Exampl e 4 | Compar ative Example 1 | Compar ative Example 2 | Compar ative Example 3 | Compar ative Example 4 |
|---|---|---|---|---|---|---|---|---|---|---|
| Prescription feature | | | Excipie nt | Excipie nt | Excipie nt | Excipie nt | Excipien t | Excipien t | Excipien t | Excipien t |
| | | | MCC | MCC+ Manitol | MCC+L -HPC | MCC+D CP | MCC+M anitol | MCC+M anitol | MCC+M anitol | MCC+M anitol |
| | | | Lubrica nt | Lubrica nt | Lubrica nt | Lubrica nt | Lubrican t | Lubrican t | Lubrican t | Lubrican t |
| | | | PRUV ^{®} | PRUV ^{®} | PRUV ^{®} | PRUV^{®} | GMS | Mg-Stearate | Ca-Stearate | Sucrose Stearate |
| Weighing ↓ Sieving and mixing ↓ Compacti ng ↓ Sizing | 1 | Sitagliptin Phosphate hydrate | 128.5 | | | | | | | |
| | 2 | Dapagliflozin L-Proline | 15.6 | | | | | | | |
| | 3 | Microcrystalline Cellulose (MCC) | 239.6 | 139.6 | | | | | | |
| | 4 | D-mannitol | - | 100.0 | - | - | 100.0 | | | |
| | 5 | L-HPC (Low-Substituted Hydroxypropyl Cellulose) | - | | 100.0 | - | - | - | - | - |
| | 6 | Dicalcium Phosphate Anhydrous(DCP) | - | - | - | 100.0 | - | - | - | - |
| | 7 | Croscarmel lose Na | 8.3 | | | | | | | |
| | 8 | Sodium stearyl fumarate(PRUV^{®}) | 14.0 | | | | - | - | - | - |
| | 9 | Glyceryl Monotearate(GMS) | - | | - | - | 14.0 | - | - | |
| | 10 | Mg-Stearate | - | - | - | - | - | 14.0 | - | |
| | 11 | Ca-Stearate | - | - | - | - | - | - | 14.0 | |
| | 12 | Sucrose Stearate | - | - | - | - | - | - | - | 14.0 |
| Final mixing | 13 | Sodium stearyl fumarate(PRUV^{®} ) | 14.0 | | | | - | - | - | - |
| | 14 | Glyceryl Monostearate | - | - | - | - | 14.0 | - | - | - |
| | 15 | Mg-Stearate | - | - | - | - | - | 14.0 | - | - |
| | 16 | Ca-Stearate | - | - | - | - | - | - | 14.0 | - |
| | 17 | Sucrose Stearate | - | - | - | - | - | - | - | 14.0 |
| Total (mg) | | | 420.0 | | | | | | | |

The results of evaluation of the total related compounds of sitagliptin during storage under accelerated conditions (40 °C and a relative humidity of 75 %) are shown in Table 7.

**[Table 7] Results of total related compound of sitagliptin (%) [Total related compound standard level: 0.2% or less]**

| | Example 1 | Example 2 | Example 3 | Example 4 | Comparative Example 1 | Comparative Example 2 | Comparative Example 3 | Comparative Example 4 |
|---|---|---|---|---|---|---|---|---|
| Initial | 0.01 | 0.01 | 0.02 | 0.02 | 0.02 | 0.01 | 0.02 | 0.02 |
| Acceleration 1M | 0.02 | 0.03 | 0.03 | 0.04 | 0.22 | 0.26 | 0.22 | 0.24 |
| Acceleration 3M | 0.05 | 0.06 | 0.05 | 0.09 | 0.62 | 0.81 | 0.73 | 0.82 |

The results of evaluation of the total related compounds of dapagliflozin during storage under accelerated conditions (40 °C and a relative humidity of 75 %) are shown in Table 8.

**[Table 8] Results of total related compound of dapagliflozin (%) [Total related compound standard level: 2.0% or less]**

| | Example 1 | Example 2 | Example 3 | Example 4 | Comparative Example 1 | Comparative Example 2 | Comparative Example 3 | Comparative Example 4 |
|---|---|---|---|---|---|---|---|---|
| Initial | 0.04 | 0.05 | 0.05 | 0.05 | 0.05 | 0.05 | 0.05 | 0.05 |
| Acceleration 1M | 0.23 | 0.22 | 0.26 | 0.92 | 0.42 | 0.53 | 0.48 | 0.58 |
| Acceleration 3M | 0.68 | 0.73 | 0.75 | 2.30 | 1.62 | 1.89 | 1.79 | 1.97 |

The results of evaluation of the total related compounds of sitagliptin during storage under stress conditions (60 °C) are shown in Table 9.

**[Table 9] Results of total related compound of sitagliptin (%) [Total related compound standard level: 0.2% or less]**

| | Example 1 | Example 2 | Example 3 | Example 4 | Comparative Example 1 | Comparative Example 2 | Comparative Example 3 | Comparative Example 4 |
|---|---|---|---|---|---|---|---|---|
| Initial | 0.02 | 0.01 | 0.01 | 0.02 | 0.02 | 0.01 | 0.02 | 0.02 |
| Stress 1M | 0.11 | 0.12 | 0.13 | 0.14 | 0.28 | 0.38 | 0.33 | 0.35 |
| Stress3M | 0.14 | 0.16 | 0.18 | 0.18 | 0.85 | 1.18 | 1.02 | 1.21 |

The results of evaluation of the total related compounds of dapagliflozin during storage under stress conditions (60 °C) are shown in Table 10.

**[Table 10] Results of total related compound of dapagliflozin (%) [Total related compound standard level: 2.0% or less**

| | Example 1 | Example 2 | Example 3 | Example 4 | Comparative Example 1 | Comparative Example 2 | Comparative Example 3 | Comparative Example 4 |
|---|---|---|---|---|---|---|---|---|
| Initial | 0.05 | 0.05 | 0.04 | 0.05 | 0.05 | 0.05 | 0.05 | 0.05 |
| Stress 1M | 0.24 | 0.26 | 0.33 | 1.11 | 0.51 | 0.60 | 0.54 | 0.66 |
| Stress 3M | 0.98 | 0.99 | 1.08 | 2.75 | 1.97 | 2.25 | 2.16 | 2.42 |

According to the results of Tables 7-10, when sodium stearyl fumarate (PRUV^{®}) was used as the lubricant (Examples 1-3), the standard levels of related compounds were satisfied, indicating that sodium stearyl fumarate (PRUV^{®}) is an appropriate lubricant that does not impair stability. However, according to Comparative Examples 1-4, it was confirmed that the other lubricants glyceryl monostearate, magnesium stearate, calcium stearate, and sucrose stearate did not meet the standard levels under the accelerated and stress conditions.

In addition, when microcrystalline cellulose, D-mannitol, and/or low-substituted hydroxypropyl cellulose were used as excipients, along with sodium stearyl fumarate (PRUV^{®}), the stability of related compounds was ensured (Examples 1-3). However, when dicalcium phosphate anhydrous was used as excipient along with sodium stearyl fumarate (PRUV^{®}) (Example 4), the standard level of related compounds of dapagliflozin under accelerated and stress conditions was not satisfied.

### Test example 4: Evaluation of productivity according to lubricant amount

In Test example 4, productivity according to the amount of lubricant was evaluated with sodium stearyl fumarate (PRUV^{®}) and microcrystalline cellulose, D-mannitol, and low-substituted hydroxypropyl cellulose, of which stability was secured. In addition, after completion of sample preparation, stability evaluation was carried out under accelerated and stress conditions. The sample preparation was carried out according to the same method as in Test Example 3.

**[Table 11] Prescriptions with different lubricant amounts [units: mg]**

| Proce ss | N ο | Excipient | Compa rative Exampl e 5 | Examp le 5 | Exam ple 6 | Compar ative Exampl e 6 | Compar ative Exampl e 7 | Exampl e 7 | Exampl e 8 | Compar ative Exampl e 8 |
|---|---|---|---|---|---|---|---|---|---|---|
| Prescription feature | | | Excipient: MCC+mannitol PRUV ^{®} quantity prescription | | | | Excipient: MCC+L-HPC PRUV^{®} quantity prescription | | | |
| Weighi ng ↓ Sievin g and mixing ↓ Comp acting ↓ Sizing | 1 | Sitagliptin Phosphate hydrate | 128.5 | | | | | | | |
| | 2 | Dapagliflozi n L-Proline | 15.6 | | | | | | | |
| | 3 | Microcrystall ine Cellulose (MCC) | 159.2 | 155.0 | 134.0 | 121.4 | 159.2 | 155.0 | 134.0 | 121.4 |
| | 4 | D-mannitol | 100.0 | | | | - | - | - | - |
| | 5 | L-HPC (Low-Substituted Hydroxypro pyl Cellulose) | - | - | - | - | 100.0 | | | |
| | 6 | Croscarmell ose Na | 8.3 | | | | | | | |
| | 7 | Sodium stearyl fumarate (PRUV^{®}) | 4.2 | 8.4 | 25.2 | 37.8 | 4.2 | 8.4 | 25.2 | 37.8 |
| Final mixing | 1 3 | Sodium stearyl fumarate (PRUV^{®}) | 4.2 | 4.2 | 8.4 | 8.4 | 4.2 | 4.2 | 8.4 | 8.4 |
| Total (mq) | | | 420.0 | | | | | | | |

The appearance of the tablets of Examples 5 and Comparative Example 5 after tableting were photographed, and the time required for discharging granules during tableting into each tablet was measured. The results are shown in FIG. 3. "The time required for discharging granules during tableting" above refers to the time it takes during tableting for all the granules filled in a feeder to be compressed into tablets and for all of the granules inside the feeder to be exhausted. This test was evaluated based on an amount of 420 g of granules filled in the feeder.

### Test example 5: Evaluation of stability according to lubricant amount

For the samples prepared according to Table 11, the total related compounds of sitagliptin in tablets according to lubricant amount during storage under accelerated conditions (40 °C and 75 % relative humidity) was evaluated. The results are shown in Table 12.

**[Table 12]**

| Evaluation of related compound under accelerated conditions according to amount of sodium stearyl fumarate (PRUV^{®}) | | | | | | | | |
|---|---|---|---|---|---|---|---|---|
| Total related compound of sitagliptin (%) [Total related compound standard level: 0.2% or less] | | | | | | | | |
| Sitaglipti n | Compa rative Exampl e5 | Exampl e 5 | Exampl e 6 | Compa rative Exampl | Compa rative Exampl e 6 e 7 | Exampl e 7 | Exampl e 8 | Compa rative Exampl e8 |
| Initial | 0.01 | 0.01 | 0.01 | 0.01 | 0.01 | 0.02 | 0.02 | 0.01 |
| Acceler ation 1M | 0.02 | 0.02 | 0.03 | 0.02 | 0.02 | 0.02 | 0.03 | 0.02 |
| Acceler ation 3M | 0.04 | 0.05 | 0.05 | 0.07 | 0.04 | 0.04 | 0.05 | 0.06 |

The results of evaluation of the total related compound of dapagliflozin in tablets according to lubricant amount during storage under accelerated conditions (40 °C and 75 % relative humidity) are shown in Table 13.

**[Table 13]**

| Evaluation of related compound under accelerated conditions according to amount of sodium stearyl fumarate (PRUV^{®}) | | | | | | | | |
|---|---|---|---|---|---|---|---|---|
| Total related compound of dapagliflozin (%) [Total related compound standard level: 2.0% or less] | | | | | | | | |
| Dapaglifl ozin | Compar ative Example 5 | Exam ple 5 | Exam ple 6 | Compar ative Example 6 | Compar ative Example 7 | Exam ple 7 | Exam ple 8 | Compar ative Example 8 |
| Initial | 0.05 | 0.04 | 0.05 | 0.04 | 0.04 | 0.03 | 0.05 | 0.05 |
| Accelera tion 1M | 0.21 | 0.24 | 0.25 | 0.37 | 0.23 | 0.22 | 0.28 | 0.35 |
| Accelera tion 3M | 0.41 | 0.58 | 0.75 | 0.88 | 0.45 | 0.55 | 0.80 | 0.82 |

The results of evaluation of the total related compound of sitagliptin in tablets according to lubricant amount during storage under stress conditions (60 °C) are shown in Table 14.

**[Table 14] Evaluation of related compound under stress conditions according to amount of sodium stearyl fumarate (PRUV^{®})**

| Total related compound of sitagliptin (%) [Total related compound standard level: 0.2% or less] | | | | | | | | |
|---|---|---|---|---|---|---|---|---|
| Sitaglip tin | Compar ative Exampl e5 | Exampl e 5 | Exampl e 6 | Compar ative Exampl e 6 e 7 | Compar ative Exampl | Exampl e 7 | Exampl e 8 | Compar ative Exampl e8 |
| Initial | 0.01 | 0.02 | 0.01 | 0.02 | 0.02 | 0.02 | 0.01 | 0.02 |
| Stress 1M | 0.04 | 0.07 | 0.06 | 0.09 | 0.03 | 0.06 | 0.07 | 0.08 |
| Stress 3M | 0.08 | 0.09 | 0.08 | 0.17 | 0.06 | 0.07 | 0.09 | 0.16 |

The results of evaluation of the total related compound of dapagliflozin in tablets according to lubricant amount during storage under stress conditions (60 °C) are shown in Table 15.

**[Table 15] Evaluation of related compound under stress conditions according to amount of sodium stearyl fumarate (PRUV^{®})**

| Total related compound of dapagliflozin (%) [Total related compound standard level: 2.0% or less] | | | | | | | | |
|---|---|---|---|---|---|---|---|---|
| Dapagliflozin | Compar ative Exampl e5 | Exampl e 5 | Exampl e 6 | Compar ative Exampl e 6 e 7 | Compar ative Exampl | Exampl e 7 | Exampl e 8 | Compar ative Exampl e8 |
| Initial | 0.04 | 0.04 | 0.05 | 0.05 | 0.04 | 0.04 | 0.04 | 0.05 |
| Acceleration 1M | 0.14 | 0.24 | 0.28 | 0.62 | 0.17 | 0.23 | 0.31 | 0.57 |
| Acceleration 3M | 0.48 | 0.54 | 0.78 | 1.82 | 0.53 | 0.61 | 0.82 | 1.77 |

### Test example 6: Evaluation of dissolution rate according to lubricant amount

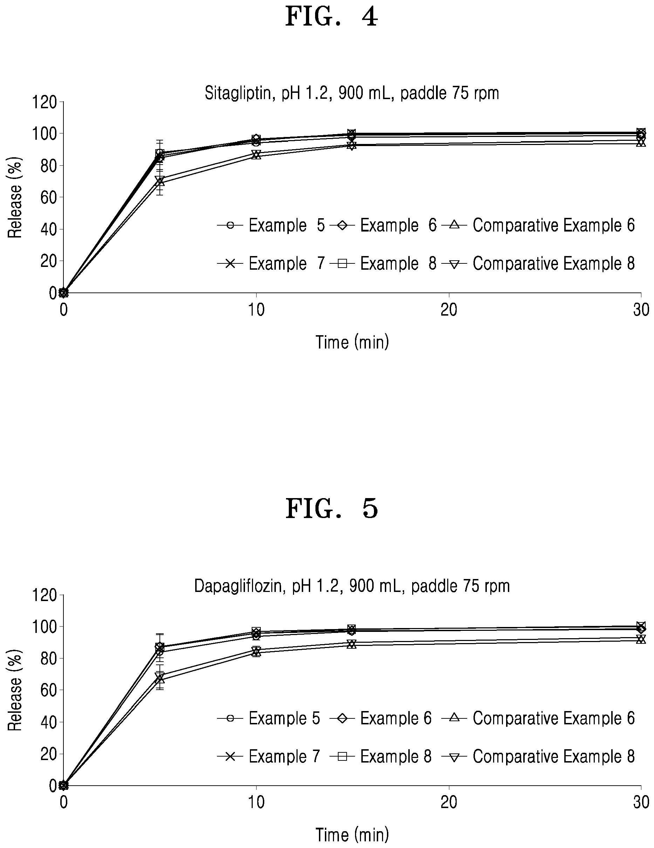

For the samples prepared according to Table 11, dissolution rate evaluation was carried out, and the results are shown in FIGS. 4 and 5.

FIG. 4 is a graph showing the results of a sitagliptin dissolution test of Examples 5 to 8 and Comparative Examples 6 and 8.

FIG. 5 is a graph showing the results of a dapagliflozin dissolution test of Examples 5 to 8 and Comparative Examples 6 and 8.

In addition, the amounts of the active ingredients of Examples 5-8 and Comparative Examples 6 and 8 were evaluated. The results are shown in Tables 16 and 17.

**[Table 16] Sitagliptin content results**

| Content (n=3) | Example 5 | Example 6 | Comparative Example 6 | Example 7 | Example 8 | Comparative Example 8 |
|---|---|---|---|---|---|---|
| Content ± standard deviation | 99.8 ± 0.4 | 101.2 ±1.2 | 100.7 ± 0.7 | 99.6 ± 1.0 | 99.5 ± 1.8 | 101.7 ± 1.5 |

**[Table 17] Dapagliflozin content results**

| Content (n=3) | Example 5 | Example 6 | Comparative Example 6 | Example 7 | Example 8 | Comparative Example 8 |
|---|---|---|---|---|---|---|
| Content ± standard deviation | 99.9 ± 0.6 | 100.5 ± 0.8 | 100.2 ± 1.3 | 100.4 ± 1.4 | 101.4 ± 0.7 | 100.5 ± 1.5 |

According to the test result of Test Example 4, when the content of sodium stearyl fumarate did not reach 3% of the total weight of the tablet (Comparative Examples 5 and 7), the amount of lubricant was insufficient, resulting in tableting defects. In addition, it was confirmed that productivity was lowered because during the tableting process, the time required for discharging granules during tableting was delayed. However, when the content of sodium stearyl fumarate was 3% or more of the total weight of the tablet (Examples 5-8 and Comparative Examples 6 and 8), tableting into tablets was possible without tableting defects. Referring to FIG. 3, for Example 5, in which the content of sodium stearyl fumarate was 3% or more of the total weight of the tablet, it was possible to form tablets smoothly without tableting defects, with a significantly shorter time required for discharging mixture powder, compared to Comparative Example 5, in which the content of sodium stearyl fumarate was less than 3% of the total weight of the tablet.

According to the test results of Test Example 5, in Comparative Examples 5-8 and Examples 5-8, the stability of related substances which conformed to the standard level was ensured under accelerated and stress conditions. However, Comparative Example 6 and Comparative Example 8 having a relatively high lubricant ratio, showed a tendency for the related compounds of both sitagliptin and dapagliflozin to increase under stress conditions to the standard levels.

According to the test results of Test Example 6, it was confirmed that when sodium stearyl fumarate was present in more than 8% of the total weight of the tablet, the dissolution rate of the active ingredients was reduced. Specifically, in comparison of dissolution patterns, Comparative Examples 6 and 8 showed a decrease in dissolution rate, as compared with those of Examples 6 and 8 (see FIGS. 4 and 5). In addition, according to the content evaluation result of Test Example 6, it was confirmed that there was no decrease in the content of the active ingredients in each tablet (see Tables 16 and 17). Taken together, it was determined that the dissolution rate decreased due to the over-lubrication of the granules.

### Test example 7: Formulation uniformity test

The contents per tablet of Examples 9 and 10 are as in Table 18. The preparation procedures were as follows.

**[Table 18]**

| | Example 9 | Example 10 |
|---|---|---|
| 1. Sitagliptin phosphate hydrate | 128.5 mg | 128.5 mg |
| 2. Dapagliflozin L-proline | 15.6 mg | 15.6 mg |
| 3. Microcrystalline cellulose (Av PH102) | 117.6 mg | 117.6 mg |
| 4. D-mannitol | 102.0 mg | 102.0 mg |
| 5.Croscarmelose sodium | 8.3 mq | 8.3 mq |
| 6.Sodium stearyl fumarate | 20.0 mg | 20.0 mg |
| 7.Sodium stearyl fumarate | 8.0 mg | 8.0 mg |
| Total | 400 mg | |

### Preparation of Example 9

(1) Each ingredient was weighed to be an amount of 1000T. (2) All the ingredients except for 28.0 mg of sodium stearyl fumarate (PRUV) was sieved through a 30-mesh sieve. (3) Powder having passed through the sieve was mixed with a Bin mixer at 17 rpm for 30 min. (4) Sodium stearyl fumarate (PRUV) and the resulting product prepared in step (3) were mixed with a Bin mixer at 17 rpm for 5 min. (5) Tableting was performed with a tablet hardness of 12-14kp using an AutoTab-200TR (Ichihachi Seiki Co., Ltd, Japan) with a rectangular punch having a width of 12.8 mm and a length of 7.0 mm.

### Preparation of Example 10

(1) Each ingredient was weighed to be an amount of 1000T. (2) All the ingredients except for 8.0 mg of sodium stearyl fumarate (PRUV) was sieved through a 30-mesh sieve. (3) Powder having passed through the sieve was mixed with a Bin mixer at 17 rpm for 30 min. (4) Pressed flakes were formed using a roller compactor at a roll rpm of 3.0 and a screw rpm of 35.0 at a hydraulic pressure of 2.5 Mpa. (5) The flakes prepared in step (4) were sieved using an oscillator with a 20-mesh. (6) The remainder of 8.0 mg of Sodium stearyl fumarate (PRUV) and the resulting product prepared in step (5) were mixed with a Bin mixer at 17 rpm for 5 min. (7) Tableting was performed with a tablet hardness of 12-14 kp using an AutoTab-200TR (Ichihachi Seiki Co., Ltd, Japan) with a rectangular punch having a width of 12.8 mm and a length of 7.0 mm.

For future comparison of appearance, a coating process was performed at a product temperature of 40 °C using a PVA-based film coating agent with the product name of Opadry in an amount equivalent to about 3% of the total mass of the tablet (Example 13).

### Preparation of Example 11 and Example 12

The contents per tablet of Examples 11 and 12 are as in Table 19. The preparation procedures were as follows.

**[Table 19]**

| | Example 11 | Example 12 |
|---|---|---|
| 1. Sitagliptin phosphate hydrate | 128.5 mg | 128.5 mg |
| 2. Dapagliflozin L-proline | 15.6 mg | 15.6 mg |
| 3. Microcrystalline cellulose (Av PH102) | 60.8 mg | 60.8 mg |
| 4. Microcrystalline cellulose (Av PH102) | 56.8 mg | 56.8 mg |
| 5. D-mannitol | 102.0 mg | 102.0 mg |
| 6.Croscarmelose sodium | 4.0 mg | 4.0 mg |
| 7.Croscarmelose sodium | 4.3 mg | 4.3 mg |
| 8.Sodium stearyl fumarate | 20.0 mg | 20.0 mg |
| 9.Sodium stearyl fumarate | 8.0 mg | 8.0 mg |
| Total | 400 mg | |

### Preparation of Example 11

(1) Each ingredient was weighed to be an amount of 1000T. (2) 128.5 mg of sitagliptin phosphate hydrate, 60.8 mg of microcrystalline cellulose, 102.0 mg of D-mannitol, 4.0 mg of croscarmelose sodium, and 20.0 mg of sodium stearyl fumarate were sieved through a 30-mesh sieve. (3) Powder having passed through the sieve was mixed with a Bin mixer at 17 rpm for 30 min. (4) Pressed flakes were formed using a roller compactor at a roll rpm of 3.0 and a screw rpm of 35.0 at a hydraulic pressure of 2.5 Mpa. (5) The flakes prepared in step (4) were sized using an oscillator with a 20-mesh. (6) The remainder 15.6 mg of dapagliflozin L-proline, 56.8 mg of microcrystalline cellulose, 4.3 mg of croscarmellose sodium, 8.0 mg of sodium stearyl fumarate, and the resulting product prepared in step (5) were mixed with a Bin mixer at 17 rpm for 5 min. (7) Tableting was performed with a tablet hardness of 12-14kp using an AutoTab-200TR (Ichihachi Seiki Co., Ltd, Japan) with a rectangular punch having a width of 12.8 mm and a length of 7.0 mm.

### Preparation of Example 12

(1) Each ingredient was weighed to be an amount of 1000T. (2) 15.6 mg of dapagliflozin L-proline, 60.8 mg of microcrystalline cellulose, 102.0 mg of D-mannitol, 4.0 mg of croscarmelose sodium, and 20.0 mg of sodium stearyl fumarate were sieved through a 30-mesh sieve. (3) Powder having passed through the sieve was mixed with a Bin mixer at 17 rpm for 30 min. (4) Pressed flakes were formed using a roller compactor at a roll rpm of 3.0 and a screw rpm of 35.0 at a hydraulic pressure of 2.5 Mpa. (5) The flakes prepared in step (4) were sized using an oscillator with a 20-mesh. (6) 128.5 mg of sitagliptin phosphate hydrate, 56.8 mg of microcrystalline cellulose, 4.3 mg of croscarmellose sodium, 8.0 mg of sodium stearyl fumarate, which were excluded in step (2), and the resulting product prepared in step (5) were mixed with a Bin mixer at 17 rpm for 5 min. (7) Tableting was performed with a tablet hardness of 12-14 kp using an AutoTab-200TR (Ichihachi Seiki Co., Ltd, Japan) with a rectangular punch having a width of 12.8 mm and a length of 7.0 mm.

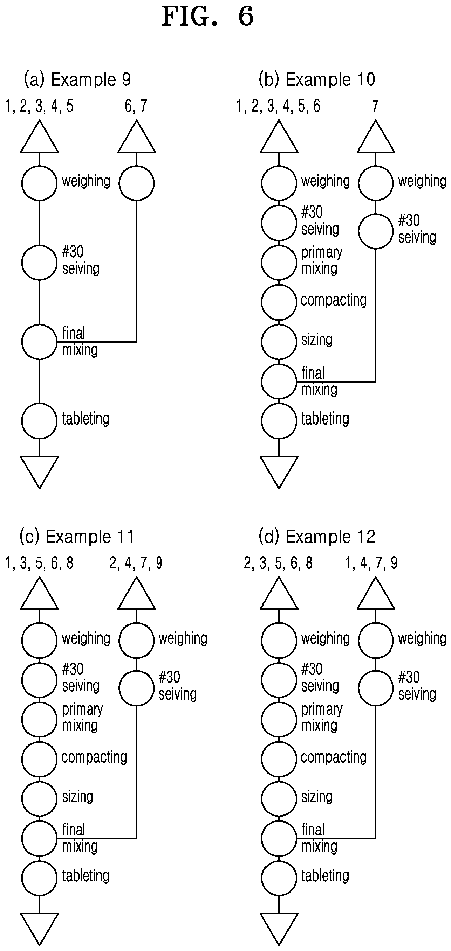

FIG. 6 is a process chart illustrating a process of preparing composite tablets of Examples 9 to 12. The numbers shown in FIG. 6 represent the numbers of the ingredients shown in Table 18 (Examples 9 and 10) and Table 19 (Examples 11 and 12). The tablets of Example 9 were prepared according to a direct compression method, and the composite formulations of Examples 10-12 were prepared by roller compaction.

### Content uniformity test

### Sample preparation

20 Tablets were taken from the tablets prepared in each of Examples 9-12 and ground, and an amount equivalent to 10 tablets was taken and put into a 500-mL volumetric flask. 300 mL was added thereto and stirred with a magnetic bar for 60 minutes to complete dissolution, and then the magnetic bar was removed, followed by filling with a diluent to a marked line. 4 mL of this solution was taken and put into a 50-mL volumetric flask, adjusted with a diluent to a marked line, and then filtered with a 0.45 µm membrane filter to use as a sample solution. Testing was carried out under the following conditions.

### Mobile phase

pH 2.0 buffer: acetonitrile (ACN) = 15 : 5 (v/v)

### Diluent

Same as mobile phase

### HPLC conditions

Column: 15 cm × 4.6 mm, 5 µm CN or equivalent column
Pump: 1.0 mL/min
Injection Vol.: 20 µL
UV lamp: 205 nm
Analysis time: 30 min

The results of the formulation uniformity test of the tablets prepared in Examples 9-12 are shown in the tables below. In assessment according to the assessment method of the content uniformity test among the formulation uniformity tests of general test methods in the 11th revision of the Korean Pharmacopoeia, it was judged to be acceptable when the assessment value was 15 % or less (n=10).

**[Table 20]**

| Sitagliptin | Example 9 | Example 10 | Example 11 | Example 12 |
|---|---|---|---|---|
| Average (%) | 98.2 | 101.2 | 100.4 | 99.8 |
| Standard deviation | 6.2 | 1.2 | 2.1 | 2.3 |
| Assessment value | 14.9 | 2.9 | 5.1 | 5.5 |

**[Table 21]**

| Dapagliflozin | Example 9 | Example 10 | Example 11 | Example 12 |
|---|---|---|---|---|
| Average (%) | 97.8 | 99.2 | 98.8 | 101.0 |
| Standard deviation | 7.8 | 2.3 | 3.3 | 3.4 |
| Assessment value | 18.7 | 5.6 | 7.8 | 8.2 |

As shown in Tables 20 and 21, for the tablets prepared by a direct compression method according to Example 9, it can be found that the content uniformity of the tablets exceeds or approximates to the standard levels. This occurs due to the low density properties (small particle size and large volume) of sitagliptin and dapagliflozin. This is expected since, according to the preparation method of Example 9, the flowability of granules themselves is greatly reduced such that a tableting disk is not uniformly charged during tableting, and layer separation of mixed powder occurs during mixing and transfer.

In comparison, the tablets of Example 10 prepared by mixing and roller compaction of sitagliptin and dapagliflozin was found to be excellent in terms of content uniformity. The well-mixed powder was formed into lumps under strong pressure and then pulverized to a certain size, thereby improving physical properties at issue such as separation between the active ingredients and excipient, and layer separation between fine particles and large particles, and improving product quality.

For Example 12, in which a mixture portion containing dapagliflozin and the excipient was separately compacted, the loss of dapagliflozin may be small, and the sitagliptin raw material has larger particles and larger amount than dapagliflozin, and thus the content uniformity of sitagliptin seems to be superior to that of dapagliflozin. In addition, it seems that the flowability is slightly improved due to the granulation of the compacted dapagliflozin mixture portion, and thus the mass deviation also showed good results. However, the total surface area of the formulation to be lubricated by lubricants increased, and thus the surface lubrication of the final tablets appeared to be insufficient.

In addition, for Example 11, in which a mixture portion containing sitagliptin and the excipient was separately compacted, the content uniformity of both sitagliptin and dapagliflozin was slightly superior to that of Example 12, in which the mixture portion containing dapagliflozin and the excipient was separately compacted. This seems to be because dapagliflozin occupies a smaller proportion in the tablet compared to sitagliptin, and only simple mixing proceeded without a granulation process.

### Density and particle size distribution test

Bulk density and tapped density were measured using the granules prepared in Examples 11 and 12, and are shown in Table 22, and the mesh profile of the granules was measured and is shown in Table 7.

**[Table 22]**

| Sample | Example 11 | Example 12 |
|---|---|---|
| Density (Tapped/bulk) | 0.65/0.44 g/mL | 0.85/0.65 g/mL |
| Hausner ratio | 1.47 | 1.31 |

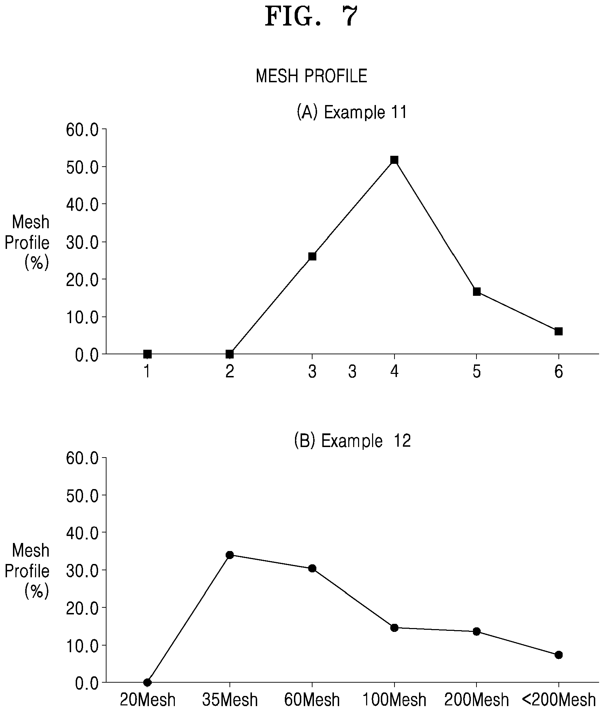

As shown in Table 22, hausner ratios (tapped density/bulk density) were measured from bulk density and tapped density values measured for Examples 11 and 12. As a result, it is found that that Example 12 has a smaller hausner ratio value than that of Example 11, thus exhibiting good flowability and improving tableting properties. In addition, as shown in FIG. 7, it can be seen that the sample of Example 12 has a more uniform particle size distribution than the sample of Example 11. It can be confirmed that compared to Example 11, Example 12 has more granules of 30-60 meshes, thus having a larger particle size with a smaller particle size deviation.

### Test example 8: Tablet size measurement test

### Preparation of Example 13

A composite formulation of Example 13 was prepared in the same content and sequence as in Example 10. For comparison of appearance, a coating process was performed at a product temperature of 40 °C using a PVA-based film coating agent with the product name of Opadry in an amount equivalent to about 3% of the total mass of the tablet.

### Tablet size measurement

The width, length, thickness, and mass of the tablets prepared in Example 13, JANUVIA tablets and FORXIGA tablets, were measured. FIG. 8 shows a photographic image of (a) JANUVIA 100 mg (100 mg of sitagliptin), (b) FORXIGA 10 mg (12.3 mg of dapagliflozin propanediol hydrate), and (c) the tablet prepared in Example 13.

**[Table 23]**

| Sample | JANUVIA (circular) | FORXIGA (diamond) | Example 13 (rectangular) |
|---|---|---|---|
| Width | 10 mm | 11 mm | 13 mm |
| Length | 10 mm | 8 mm | 7 mm |
| Thickness | 4.3 mm | 4.2 mm | 5.0 mm |
| Mass | 412 mg | 260 mg | 410 mg |

As shown in Table 23 and FIG. 8, for Example 13, while simultaneously including 100 mg of sitagliptin and 10 mg of dapagliflozin, the size of the tablets was not visually or numerically significantly larger than that of the other two tablets. In addition, for Example 13, the width increased by about 30% compared to the circular tablet of JANUVIA, but the length decreased by about 30%, and had a similar area. Example 13 has a smallest cross-sectional area among the three tablets, and is designed so as to pass through the cross-section of the throat when swallowing. Through these, it can be found that the size, weight, and cross-sectional area at swallowing of the drug were dramatically improved, compared to taking two tablets of JANUVIA tablet or FORXIGA tablet at the same time.

Those skilled in the art will recognize that the present invention may be embodied in other specific forms without departing from the spirit or essential features thereof. The described embodiments are for illustrative purposes only in all respects and not restrictive. The scope of the invention is therefore indicated by the appended claims rather than by the foregoing description. All modifications within the meaning and scope equivalent to the claims are to be embraced within the scope of the present invention.

## Claims

1. A composite formulation comprising:
sitagliptin or a pharmaceutically acceptable salt thereof, or a hydrate thereof; and
dapagliflozin or a pharmaceutically acceptable salt thereof, or a hydrate thereof,
wherein the composite formulation includes, as a lubricant, sodium stearyl fumarate, magnesium stearate, or a combination thereof.

2. The composite formulation of claim 1, comprising sodium stearyl fumarate as the lubricant.

3. The composite formulation of claim 1, wherein the sitagliptin or the pharmaceutically acceptable salt thereof, or the hydrate thereof, includes sitagliptin phosphate hydrate.

4. The composite formulation of claim 1, wherein the dapagliflozin or the pharmaceutically acceptable salt thereof, or the hydrate thereof, includes dapagliflozin L-proline.

5. The composite formulation of claim 1, wherein the sodium stearyl fumarate is in an amount of about 3-8 wt% with respect to a total weight of the composite formulation.

6. The composite formulation of claim 1, comprising an excipient selected from microcrystalline cellulose (MCC), mannitol, pregelatinized starch, low-substituted hydroxypropyl cellulose (L-HPC), crospovidone, cross-linked sodium carboxymethlcellulose (cross-linked CMC Na), and mixtures thereof.

7. The composite formulation of claim 1, wherein the composite formulation is in the form of tablets, capsules, or granules.

8. The composite formulation of claim 1, wherein the composite formulation comprises compacted granules including: the sitagliptin or the pharmaceutically acceptable salt thereof, or the hydrate thereof; the dapagliflozin or the pharmaceutically acceptable salt thereof, or the hydrate thereof; and sodium stearyl fumarate.

9. The composite formulation of claim 1, wherein the composite formulation has a form in which a width and length are each about 5-15 mm and a thickness is about 3-8 mm.

10. The composite formulation of claim 1, wherein the sitagliptin or the pharmaceutically acceptable salt thereof, or the hydrate thereof, is in an amount of about 10-40 wt% with respect to a total weight of the composite formulation.

11. The composite formulation of claim 1, wherein the dapagliflozin or the pharmaceutically acceptable salt thereof, or the hydrate thereof, is in an amount of about 2-10 wt% with respect to a total weight of the composite formulation.

12. The composite formulation of claim 1, further comprising metformin or a pharmaceutically acceptable salt thereof, or a hydrate thereof.

13. A method of preparing the composite formulation according to any one of claims 1 to 12, the method comprising:
preparing a mixture portion including sitagliptin or a pharmaceutically acceptable salt thereof, or a hydrate thereof, dapagliflozin or a pharmaceutically acceptable salt thereof, or a hydrate thereof, an excipient, and a lubricant;
granulating the mixture portion; and
further adding a lubricant to the granulated material and mixing together.

14. A method of preparing the composite formulation according to any one of claims 1 to 12, the method comprising:
preparing a first mixture portion including sitagliptin or a pharmaceutically acceptable salt thereof, or a hydrate thereof, an excipient, and a lubricant;
preparing a second mixture portion including dapagliflozin or a pharmaceutically acceptable salt thereof, or a hydrate thereof, an excipient, and a lubricant;
separately granulating the first mixture portion and/or the second mixture portion; and
mixing obtained granulated material with a non-granulated mixture portion and an additional lubricant.

15. The method of claim 13, wherein the granulation comprises forming a compact product by using a roller compactor.

16. The method of claim 13, further comprising tableting the mixture mixed with the additional lubricant.
